# FASCICULE DE BREVET EUROPEEN

(11) **EP 1 711 460 B1**
(45) Date de publication et mention de la délivrance du brevet: **02.06.2010**
(21) Numéro de dépôt: 05717563.0
(22) Date de dépôt: 04.02.2005
(51) Int. Cl.: C07C 319/04, C07C 321/04, C08F 212/08, C07C 319/24

(54) **MELANGE DE DODECANETHIOLS, SON PROCEDE DE FABRICATION ET SES UTILISATIONS**
MISCHUNG VON DODECANTHIOLEN, VERFAHREN ZU DEREN HERSTELLUNG UND DEREN VERWENDUNG
MIXTURE OF DODECANETHIOLS, METHOD FOR THE PRODUCTION AND USE THEREOF

(30) Priorité: 06.02.2004 FR 0401154
(43) Date de publication de la demande: 18.10.2006
(73) Titulaire: ARKEMA FRANCE, 92700 Colombes (FR)
(72) Inventeur: PIRRI, Rosangela, F-64121 Montardon (FR); FREMY, Georges, F-64390 Sauveterre de Bearn (FR)
(74) Mandataire: Lhoste, Catherine
(86) Numéro de dépôt international: PCT/FR2005/000260
(87) Numéro de publication internationale: WO 2005/082846

(56) Documents cités:
- EP-A- 0 269 517
- FR-A- 2 082 724
- FR-A- 2 531 426

## Description

La présente invention concerne un mélange de dodécanethiols son procédé de fabrication et ses utilisations notamment comme agent de transfert de chaîne dans les réactions de polymérisation radicalaire.

On connaît par la demande FR 2531426 un procédé de préparation de tertio-alkyl mercaptans, notamment de tertio-dodécylmercaptans, par l'action de l'hydrogène sulfuré sur une oléfine. Ce document décrit de façon précise la réalisation du procédé mis en oeuvre sur le plan industriel et utilisant le tétrapropylène comme matière première afin de synthétiser le tertiododecylmercaptan ci-après dénommé TDM 4P. Ce document mentionne aussi la mise en oeuvre de ce type de procédé à partir du tri-isobutylène qui aboutit à un mélange de 2 isomères du dodécène: le di-méthyl-4,4-néopentyl-2-pentène-1 et le pentaméthyl-2,2,4,6,6-heptène-3 donnant un mélange des deux isomères de tertiododecylmercaptan correspondants dénommés ci-après TDM 3B.

Les alkyl mercaptans, et notamment les tertio-alkyl mercaptans, sont largement utilisés dans les réactions de polymérisation industrielles car ils permettent de diminuer la longueur des chaînes de polymères et d'abaisser leur masse moléculaire. Les polymères ainsi obtenus ont une viscosité plus faible, ce qui est souvent nécessaire dans certaines applications, par exemple pour une mise en forme aisée de la matière plastique par les techniques usuelles telles que le moulage par injection.

Le tétra-propylène, qui est disponible commercialement, est obtenu par oligomérisation du propène dans des conditions contrôlées de température et de pression, en présence soit d'un catalyseur acide comme des acides minéraux tels H₃PO₄ et H₂SO₄, soit d'un catalyseur organométallique tels les alkyles d'aluminium ou les métaux alcalins. Le tétra-propylène correspond à un mélange d'alcènes ayant de 10 à 14 atomes de carbone (monooléfines de C₁₀ à C₁₄), dans lequel la teneur en alcène à 12 atomes de carbone (également appelé dodécène ou oléfine en C₁₂) est comprise entre 55 et 85 %, et plus précisément entre 60 et 80% en poids. Chacun de ces alcènes est présent dans ledit mélange sous la forme d'isomères de position de la double liaison et d'isomères géométriques. Le dodécène correspond au tétramère du propène, et la présence (dans le produit de la réaction d'oligomérisation du propène conduisant au C₁₂) d'oléfines en C₁₀, C₁₁, C₁₃ et C₁₄ résulte de réactions secondaires.

Il est toujours souhaitable de disposer d'une gamme élargie de TDM afin de pouvoir mieux répondre aux besoins du marché à la fois, sur le plan technique, fonction des applications visées et sur le plan économique.

La présente invention a ainsi pour objet un mélange d'isomères du dodécanethiol préparé par réaction catalytique de l'hydrogène sulfuré sur le trimère du n-butène et présentant un diagramme de températures de distillation, à 19 millibars, tel que le point 50 est de 123 °C ± 1°C et que la différence de température entre le point 20 et le point 80 est inférieure ou égale à 4 °C.

On entend par point 20, 50 et 80 de la distillation, la température à laquelle respectivement 20, 50 et 80 % en poids du mélange sont passés en phase vapeur.

La présente invention a aussi pour objet le procédé de préparation du mélange d'isomères du dodécanethiol caractérisé en ce qu'il consiste à faire réagir l'hydrogène sulfuré sur du tri-n-butène en présence d'un catalyseur.

On entend par la dénomination « tri-n-butène » désigner un mélange de monooléfines obtenu par oligomérisation de n-butène en présence d'un système catalytique qui est souvent identique à celui mentionné précédemment. Les alcènes présents dans ledit mélange comprennent généralement de 11 à 13 atomes de carbone avec une teneur en poids en dodécène supérieure à 90 % et même à 95 % en poids. Le tri-n-butène est disponible commercialement.

De façon surprenante, il a été trouvé que le procédé selon l'invention conduit à un produit différent de ceux résultant décrits dans le brevet FR 2 531 426. En effet, le produit qui est un mélange d'isomères, présente un diagramme de températures de distillation sensiblement différent de ceux des mélanges TDM obtenus à partir du tétra-propylène ou du tri-isobutène.

Cette différence s'explique par la nature du réactif tri-n-butène utilisé qui conduit à une homogénéité de comportement des isomères du mélange bien supérieure à celles des TDM 4P. En ce qui concerne la comparaison avec les TDM 3B, on peut relever que les mélanges de l'invention ont un point 50 sensiblement supérieur au point d'ébullition du TDM qui se conduit comme un corps quasiment pur. Outre cette différence, il faut souligner que la synthèse des mélanges de l'invention, qui est sur le plan procédé très proche de celle mise en oeuvre avec les TDM 4P, évite les problèmes de stabilité thermique des produits intermédiaires que présente la synthèse des TDM 3B.

La distillation est effectuée sur une colonne Cadiot à « bande tournante » sans reflux et à une pression de 19mbars. Des mesures effectuées sur le TDM 4P et le mélange de l'invention sont données dans le tableau 1 ci-dessous.

**Tableau 1**

| **% TDM distillé** | **Températures de distillation °C** | **% mélange de l'invention distillé** | **Températures de distillation °C** |
|---|---|---|---|
| 10% | 97 | 10% | 116 |
| 20% | 106 | 19% | 122 |
| 29% | 109 | 29% | 122 |
| 40% | 113 | 40% | 123 |
| 54% | 114 | 49% | 123 |
| 64% | 118 | 59% | 123 |
| 72% | 118 | 70% | 124 |
| 81% | 121 | 83% | 125 |

Le mélange de l'invention est sur le plan industriel un mélange complexe comprenant des thiols allant de 11 à 13 atomes de carbone, mais avec une teneur en dodécanethiol -12 atomes de carbone - supérieure à 90 %, de préférence à 95 % en poids.

A titre de comparaison le mélange obtenu à partir du tétra-propylène possède un diagramme de distillation sensiblement différent en terme de niveau, le point 50 étant de 10°C inférieur ; la différence entre les points 20 et 50 est de 15 °C pour le TDM 4P à comparer aux 3 ou 4 °C des mélanges de l'invention.

Le catalyseur utilisable dans le procédé selon l'invention peut être choisi parmi un composé acide, un oxyde métallique ou une combinaison de ces 2 produits. Comme composé acide, on peut utiliser un acide de Lewis et/ou de Brönsted, solide ou liquide, miscible ou non dans le milieu de réaction, choisi par exemple parmi un acide organique ou inorganique, une alumine, une argile, une silice ou une silice-alumine, une zéolithe, un hétéropolyacide ou une résine échangeuse de cations faiblement ou fortement acide. Parmi les métaux dont un oxyde peut être utilisé comme catalyseur, on peut citer le chrome, le cobalt, le molybdène, le tungstène, le zirconium, le niobium ou le nickel.

On préfère utiliser comme catalyseur une résine échangeuse de cation.

Les différents polymères et copolymères à fonctions acides connus de l'homme du métier comme échangeurs de cation conviennent donc. En particulier, on peut employer des résines à base de polystyrène sulfoné, réticulées, en particulier avec du divinylbenzène, des résines acryliques ou phénylacryliques, à groupes carboxyliques libres, des résines type phénol formaldéhyde dérivé des acides phénol sulfoniques, des échangeurs lignosulfoniques. Des résines de ce genre sont commercialisées sous différentes dénominations.

De manière encore plus préférentielle on utilise une résine sulfonée de copolymère styrène-divinylbenzène, disponible dans le commerce sous la dénomination Amberlyst^{®} 15.

Le ratio molaire de l'hydrogène sulfuré par rapport à l'oléfine est au minimum de 1 (correspondant à la stoechiométrie), et est généralement compris entre 1 et 100, de préférence entre 1 et 20, encore plus préférentiellement entre 1 et 5.

La température de mise en oeuvre du procédé selon l'invention dépend du catalyseur utilisé et est généralement comprise entre 10 et 250 °C, de façon préférée entre 50 et 150 °C, et encore plus préférée entre 70 et 120°C. La pression peut être égale ou supérieure à la pression atmosphérique. Elle est généralement comprise entre 5 et 80 bars et, de façon préférée, entre 10 et 50 bars, et encore plus préférée entre 10 et 20 bars.

Le procédé peut être mené de façon continue ou discontinue. De façon préférée, il est mené en continu.

La vitesse volumique horaire, définie comme étant le ratio entre le débit volumique horaire d'oléfine sur le volume de catalyseur, dépend fortement de l'activité du catalyseur utilisé. Elle est généralement comprise entre 0,01 et 100 h⁻¹, de façon préférée entre 0,1 et 10 h⁻¹, et encore plus préférée entre 0,2 et 2 h⁻¹.

Les produits de la réaction qui peuvent comprendre les mercaptans formés, l'hydrogène sulfuré non réagi et les oléfines non converties sont séparés en utilisant toutes les méthodes conventionnelles connues par l'homme de l'art. On utilise généralement la distillation sous pression réduite pour séparer les oléfines non converties des mercaptans formés.

La présente invention a également pour objet les utilisations comme agent de transfert de chaîne dans les réactions de (co)polymérisation radicalaire ou comme matière première dans la synthèse de polysulfures.

La présente invention a également pour objet un procédé de synthèse de di-*tertio-*dodécyl polysulfures, par réaction du mélange selon l'invention avec du soufre, en présence de catalyseur basique.

Lorsque le mélange de l'invention est utilisé comme agent de transfert de chaîne dans les réactions de polymérisation radicalaire, il permet d'obtenir des polymères (ou matières plastiques) ayant une viscosité sensiblement abaissée. En outre, lorsqu'il est utilisé dans la fabrication de polysulfures, il conduit aussi à un matériau final présentant au niveau viscosité des différences substantielles. De telles propriétés sont particulièrement intéressantes au plan pratique. Elles permettent par exemple une mise en forme plus rapide de la matière plastique par les techniques usuelles de moulage par injection, autorisant ainsi une augmentation de la productivité des chaînes de production des objets moulés. Elle permet également, par exemple dans le cas d'un copolymère styrène/butadiène, de diminuer la quantité d'agent de transfert qui est nécessaire à la fabrication d'un copolymère possédant la propriété rhéologique requise, par exemple en vue de son application comme agent liant pour le couchage du papier.

La présente invention est illustrée par les exemples suivants qui sont donnés à titre purement illustratif de celle-ci et ne sauraient être utilisés pour en limiter la portée.

### Exemple 1 : Préparation du mélange à partir de tri-n-butène :

On utilise comme tri-n-butène un mélange commercial d'alcènes comprenant de 11 à 13 atomes de carbone, dont la teneur en dodécène est 98% en poids. La composition du mélange est déterminée par chromatographie en phase gaz couplée à une spectrométrie de masse avec (pour cette dernière) une technique de détection par ionisation chimique positive à l'ammoniac.

Dans un réacteur tubulaire d'une longueur de 680 mm et d'un diamètre intérieur de 27 mm, on place 200 ml de résine sulfonée de copolymère styrène-divinylbenzène (connue sous la dénomination Amberlyst^{®} 15) sèche.

Sous une pression de 15 bars, on introduit, en continu, du tri-n-butène liquide, à raison de 79 ml/h (soit 60,8 g/h ou encore 363 mmol/h) dans ce réacteur. En même temps, on y injecte 26 litres standards/h (soit 36,9 g/h ou encore 1083 mmol/h) de H₂S gazeux. Le ratio molaire H₂S sur oléfine est de 3.

Le milieu réactionnel est maintenu à la température de 90°C ± 2°C. Les réactifs sont mélangés intimement avant leur passage dans le réacteur. Le liquide s'écoulant en continu du réacteur est recueilli et l'hydrogène sulfuré restant est dégazé.

Ce liquide est soumis à une analyse par chromatographie en phase gazeuse couplée à une spectrométrie en masse. Le chromatographe utilisé est muni d'un détecteur à ionisation de flamme et d'une colonne polaire. On observe un chromatogramme comportant 2 massifs de pics, correspondant à un temps de rétention comprise entre 2,7 et 4,2 minutes d'une part, et 4,3 et 9,6 minutes d'autre part. Ces massifs sont identifiés comme correspondant respectivement au tri-n-butène et au mélange. La mesure des aires correspondant à ces 2 massifs permet après étalonnage de calculer un pourcentage en poids de tri-n-butène non transformé égal à 28% et un taux de conversion de 68%. De plus, l'absence d'autres composés montre que le taux de sélectivité en mélange est de 100%.

### Exemple 2 : Utilisation du mélange préparé à l'exemple 1 comme agent de transfert de chaîne dans la copolymérisation radicalaire du styrène et du butadiène :

Dans un réacteur de 1 litre, contenant 450 g d'eau, on introduit :
- 0,072 g de FeSO₄ et 0,19 g de sel de sodium de l'acide hydroxyméthane sulfinique (de formule : HO-CH₂-SO₂Na), correspondant à 2 des 3 composants du système amorceur
- 0,08 g d'EDTA (éthylène diamine tétracétate)
- 0,5 g de phosphate de sodium
- 43,3 g d'un mélange aqueux à 25% en poids de rosinate de potassium (émulsifiant)
- 76,94 g de styrène
- 163,59 g de butadiène
- 0,43 g de l'agent de transfert de chaîne préparé à l'exemple 1.

Les ingrédients ci-dessus sont ajoutés à l'eau à température ambiante sous agitation de manière à obtenir une émulsion.

Cette émulsion est refroidie à 9,5°C, et l'on ajoute 0,35 g d'hydroperoxyde de paramenthane (incorporé à raison de 55 % en poids dans l'eau) qui correspond au 3^{ème} composant du système amorceur.

La polymérisation démarre dès l'introduction de l'hydroperoxyde de paramenthane, et l'on poursuit l'agitation du milieu réactionnel à 150 tours/min pendant 5 heures.

La polymérisation est arrêtée en ajoutant dans le milieu le mélange comprenant 0,077 g de potasse, 0,072 g de dithiocarbanate de sodium et 0,085 g de diéthyl hydroxylamine, complété à 15 g d'eau.

Le latex obtenu a un taux de solide d'environ 19 % en poids.

Après élimination des monomères résiduels, on obtient par séchage du latex un film de copolymère styrène/butadiène d'environ 1 mm d'épaisseur.

La viscosité à l'état fondu du film est mesurée à 100°C et pour une fréquence de 1 Hz par un rhéomètre à contrainte imposée.

On obtient une valeur pour cette viscosité de 1,1. 10⁵ Pa.s.

### Exemple 3 (comparatif) : Préparation de TDM 4P à partir de tétrapropylène :

On utilise comme tétrapropylène un mélange commercial d'alcènes comprenant de 10 à 14 atomes de carbone dont la teneur en dodécène est 60 %. La composition de ce mélange est déterminée par chromatographie en phase gaz couplée à une spectrométrie de masse avec (pour cette dernière) une technique de détection par ionisation chimique positive à l'ammoniac.

On répète l'exemple 1 en remplaçant le tri-n-butène par le tétrapropylène.

On mesure un pourcentage en poids de tétrapropylène non transformé égal à 25 % et un taux de conversion de 71 %. De plus, l'absence d'autres composés montre que le taux de sélectivité en TDM est de 100%.

### Exemple 4 (comparatif) : Utilisation du TDM 4P préparé à l'exemple 3 comme agent de transfert de chaîne dans la copolymérisation radicalaire du styrène et du butadiène :

On répète l'exemple 2 en remplaçant le tertio-dodécylmercaptan préparé à l'exemple 1 par le produit obtenu à l'exemple 3.

On obtient pour la viscosité une valeur de 1,4. 10⁵ Pa.s.

### Exemple 5 : Préparation de ditertiododecyl trisulfure par réaction du TDM 4P avec le soufre en présence d'un catalyseur basique

La synthèse du ditertiododécyltrisulfure par réaction du TDM 4P avec le soufre est réalisée en présence d'un catalyseur TPS (tertiododécylmercaptan polyéthoxylé contenant 2,5% de soude)

L'appareillage est un réacteur double enveloppe en verre de 250 ml équipé d'un fritté situé avant la vanne de vidange, d'une arrivée d'azote par une canne plongeante terminée par un fritté, le débit d'azote étant contrôlé par un débimètre à bille, d'un réfrigérant à circulation d'eau relié à l'aspiration de la hotte par un compte-bulle contenant de l'huile, d'un bain thermostaté permettant la circulation d'huile dans la double enveloppe, d'un agitateur en verre terminé par une ancre en PTFE et muni d'un moteur d'agitation à tachymètre et d'une sonde thermométrique dans une gaine de verre.

Les conditions opératoires sont les suivantes :
Le réacteur est préalablement mis sous atmosphère d'azote, 2 g de catalyseur TPS (cata/RSH = 1 %) sont introduits avec de 202 g de TDM 4P (1 mol). Le réacteur est alors chauffé à 120°C. On introduit ensuite 32 g de soufre (1 mol) par portions. L'agitation est maintenue à 500 tr/min. La dissolution totale du soufre se fait en 45 minutes environ. On met alors le bullage d'azote (débit = 341/h) pendant toute la durée de la réaction.

On prend des échantillons au cours du temps (t = 0 quand on introduit le soufre) pour déterminer la teneur en mercaptan résiduel (% massique) par argentimétrie et la teneur en soufre libre par HPLC. Le pourcentage massique de mercaptan résiduel permet d'accéder à la conversion du mercaptan. La conversion du TDM 4P est de 92% au bout de 2,5 heures de réaction.

Le mélange réactionnel est ensuite refroidi à 60°C, température à laquelle on introduit 300 mmol d'oxyde d'éthylène (13,2 g) qu'on laisse réagir pendant 3 heures avec le TDM résiduel. Le milieu réactionnel est ensuite strippé sous azote. La teneur en mercaptan résiduel est inférieure à 150 ppm après réaction.

En fin de réaction, le polysulfure est filtré à chaud (40°C) sur le fritté du réacteur.

Un échantillon du produit obtenu est appelé TDM 4P S3.

### Exemple 6 : Synthèse de ditertiododécyltrisulfure par réaction du mélange de l'exemple 1 (nommé ci-après TERDAN) avec le soufre en présence de catalyseur TPS (tertiododécylmercaptan polyéthoxylé contenant 2,5% de soude)

On utilise le même mode opératoire que dans l'exemple 5 et on obtient une conversion du TERDAN équivalente à celle obtenue avec le TDM 4P lors de l'exemple 5 (92%).

Après traitement à l'oxyde d'éthylène et filtration, on isole un échantillon que nommé TERDAN S3.

### Exemple 7 : Préparation de ditertiododécylpentasulfure par réaction du TDM 4P avec le soufre en présence d'un catalyseur basique TPS.

On utilise le même appareillage que pour l'exemple 5 et on conduit la réaction comme suit. Le réacteur est préalablement mis sous atmosphère d'azote puis en présence de 1 g catalyseur TPS (cata/RSH = 0.5 %) sont introduits 202 g de TDM 4P (1 mol). Le réacteur est alors chauffé à 90°C puis on introduit ensuite 64 g de soufre (2 mol) par portions. L'agitation est maintenue à 500 tr/min. La dissolution totale du soufre se fait en 60 minutes environ. On met alors le bullage d'azote (débit = 6 1/h) pendant toute la durée de la réaction.

On opére les prélèvements d'échantillons comme dans l'exemple 5. La conversion du TDM 4P est supérieure à 99,5% au bout de 4 heures.

Le mélange réactionnel est ensuite refroidi à 60°C, température à laquelle on introduit 10 mmol d'oxyde d'éthylène (0,44 g) qu'on laisse réagir pendant 1h avec le TDM résiduel. Le milieu réactionnel est ensuite strippé sous azote. La teneur en mercaptan résiduel est inférieure à 150 ppm après réaction.

En fin de réaction, le polysulfure est filtré à chaud (40°C) sur le fritté du réacteur.

Un échantillon du produit est prélevé et nommé TDM 4P S5.

### Exemple 8 : Synthèse de ditertiododécylpentasulfure par réaction du TERDAN avec le soufre en présence de catalyseur TPS.

On répète l'exemple 7 en remplaçant le TDM 4P par le TERDAN et on obtient des résultats comparables au niveau de la conversion du TERDAN (>99.5% au bout de 4h).

Un échantillon est prélevé et nommé TERDAN S5.

On mesure alors les viscosités cinématiques des polysulfures selon la norme ASTM D445 qui sont données dans le tableau 2.

**Tableau 2**

| | **Viscosité à 40°C (mm²/s)** |
|---|---|
| Exemple 5 : TDM S3 | 65 |
| Exemple 6 : TERDAN S3 | 43 |
| Exemple 7 : TDM S5 | 121 |
| Exemple 8 : TERDAN S5 | 83 |

Ces viscosités montrent que l'on obtient des polysulfures plus fluides lorsque l'on utilise le TERDAN au lieu du TDM 4P.

## Revendications

1. Mélange d'isomères du dodécanethiol préparé par un procédé de réaction catalytique de l'hydrogène sulfuré sur le trimère du n-butène, et présentant un diagramme de températures de distillation, à 19 millibars, tel que le point 50 est de 123 °C à ± 1°C et que la différence de température entre le point 20 et le point 80 est inférieure ou égale à 4°C.

2. Mélange la revendication 1, **caractérisé en ce que** le catalyseur est choisi parmi un composé acide, un oxyde métallique ou une combinaison de ces 2 produits.

3. Mélange selon l'une des revendications 1 ou 2, **caractérisé en ce que** le catalyseur est une résine échangeuse de cation.

4. Mélange selon l'une des revendications 1 à 3, **caractérisé en ce que** le catalyseur est un copolymère de styrène sùlfoné avec du divinyl benzène

5. Mélange selon l'une des revendications 1 à 4, **caractérisé en ce que** le ratio molaire de l'hydrogène sulfuré par rapport à l'oléfine est compris entre 1 et 100, de préférence entre 1 et 20.

6. Mélange selon la revendication 5, **caractérisé en ce que** le ratio molaire de l'hydrogène sulfuré par rapport à l'oléfine est compris entre 1 et 5.

7. Mélange selon l'une des revendications 1 à 6, **caractérisé en ce que** procédé est mis en oeuvre à une température comprise entre 10 et 250°C et à une pression comprise entre 5 et 80 bars.

8. Mélange selon la revendication 7, **caractérisé en ce que** le procédé est mis en oeuvre à une température comprise entre 50 et 150°C et à une pression comprise entre 10 et 50 bars.

9. Mélange selon la revendication 8, **caractérisé en ce qu'**il est mis en oeuvre à une température comprise entre 70 et 120°C et à une pression comprise entre 10 et 20 bars.

10. Procédé de préparation des mélanges des revendications 1 à 9 **caractérisé en ce qu'**il comprend la réaction de l'hydrogène sulfuré sur du tri-n-butène en présence d'un catalyseur choisi parmi un composé acide, un oxyde métallique, une combinaison de ces deux produits et une résine échangeuse de cation.

11. Procédé de (co)polymérisation radicalaire **caractérisé en ce qu'**il est réalisé en présence du mélange selon l'une des revendications 1 à 9 utilisé comme agent de transfert de chaîne.

12. Procédé de synthèse de ditertiododécyl polysulfures **caractérisé en ce qu'**il est réalisé par réaction du mélange selon l'une des revendications 1 à 9 avec du soufre en présence d'un catalyseur basique.

## Claims

1. Mixture of isomers of dodecanethiol prepared by a process for the catalytic reaction of hydrogen sulfide with the trimer of n-butene and exhibiting a diagram of distillation temperatures, at 19 millibar, such that point 50 is 123°C ± 1°C and that the difference in temperature between point 20 and point 80 is less than or equal to 4°C.

2. Mixture according to Claim 1, **characterized in that** the catalyst is chosen from an acid compound, a metal oxide or a combination of these 2 products.

3. Mixture according to either of Claims 1 and 2, **characterized in that** the catalyst is a cation-exchange resin.

4. Mixture according to one of Claims 1 to 3, **characterized in that** the catalyst is a copolymer of sulfonated styrene with divinylbenzene.

5. Mixture according to one of Claims 1 to 4, **characterized in that** the molar ratio of the hydrogen sulfide to the olefin is between 1 and 100, preferably between 1 and 20.

6. Mixture according to Claim 5, **characterized in that** the molar ratio of the hydrogen sulfide to the olefin is between 1 and 5.

7. Mixture according to one of Claims 1 to 6, **characterized in that** the process is carried out at a temperature of between 10 and 250°C and at a pressure of between 5 and 80 bar.

8. Mixture according to Claim 7, **characterized in that** the process is carried out at a temperature of between 50 and 150°C and at a pressure of between 10 and 50 bar.

9. Mixture according to Claim 8, **characterized in that** the process is carried out at a temperature of between 70 and 120°C and at a pressure of between 10 and 20 bar.

10. Process for the preparation of the mixtures of Claims 1 to 9, **characterized in that** it comprises the reaction of hydrogen sulfide with tri(n-butene) in the presence of a catalyst chosen from an acid compound, a metal oxide, a combination of these two products and a cation-exchange resin.

11. Process for radical (co)polymerization, **characterized in that** it is carried out in the presence of the mixture according to one of Claims 1 to 9 used as chain-transfer agent.

12. Process for the synthesis of di(tert-dodecyl) polysulfides, **characterized in that** it is carried out by reaction of the mixture according to one of Claims 1 to 9 with sulfur in the presence of a basic catalyst.

## Patentansprüche

1. Dodecanthiolisomerengemisch, hergestellt nach einem Verfahren zur katalytischen Umsetzung von Schwefelwasserstoff mit n-Buten-Trimer und mit einem solchen Diagramm der Destillationstemperatur bei 19 mbar, daß der 50-Punkt 123°C ± 1°C beträgt und die Temperaturdifferenz zwischen dem 20-Punkt und dem 80-Punkt kleiner gleich 4°C ist.

2. Gemisch nach Anspruch 1, **dadurch gekennzeichnet, daß** der Katalysator unter einer sauren Verbindung, einem Metalloxid oder einer Kombination dieser beiden Produkte ausgewählt ist.

3. Gemisch nach Anspruch 1 oder 2, **dadurch gekennzeichnet, daß** es sich bei dem Katalysator um ein Kationenaustauscherharz handelt.

4. Gemisch nach einem der Ansprüche 1 bis 3, **dadurch gekennzeichnet, daß** es sich bei dem Katalysator um ein Copolymer von sulfoniertem Styrol und Divinylbenzol handelt.

5. Gemisch nach einem der Ansprüche 1 bis 4, **dadurch gekennzeichnet, daß** das Molverhältnis von Schwefelwasserstoff zu Olefin zwischen 1 und 100 und vorzugsweise zwischen 1 und 20 liegt.

6. Gemisch nach Anspruch 5, **dadurch gekennzeichnet, daß** das Molverhältnis von Schwefelwasserstoff zu Olefin zwischen 1 und 5 liegt.

7. Gemisch nach einem der Ansprüche 1 bis 6, **dadurch gekennzeichnet, daß** das Verfahren bei einer Temperatur zwischen 10 und 250°C und einem Druck zwischen 5 und 80 bar durchgeführt wird.

8. Gemisch nach Anspruch 7, **dadurch gekennzeichnet, daß** das Verfahren bei einer Temperatur zwischen 50 und 150°C und einem Druck zwischen 10 und 50 bar durchgeführt wird.

9. Gemisch nach Anspruch 8, **dadurch gekennzeichnet, daß** das Verfahren bei einer Temperatur zwischen 70 und 120°C und einem Druck zwischen 10 und 20 bar durchgeführt wird.

10. Verfahren zur Herstellung der Gemische gemäß den Ansprüchen 1 bis 9, **dadurch gekennzeichnet, daß** man Schwefelwasserstoff in Gegenwart eines unter einer sauren Verbindung, einem Metalloxid, einer Kombination dieser beiden Produkte und einem Kationenaustauscherharz ausgewählten Katalysators mit Tri-n-buten umsetzt.

11. Verfahren zur radikalischen (Co)polymerisation, **dadurch gekennzeichnet, daß** man es in Gegenwart eines Gemischs gemäß einem der Ansprüche 1 bis 9 als Kettenübertragungsmittel durchführt.

12. Verfahren zur Synthese von Di-tert.-dodecylpolysulfiden, **dadurch gekennzeichnet, daß** man es durch Umsetzung des Gemischs gemäß einem der Ansprüche 1 bis 9 mit Schwefel in Gegenwart eines basischen Katalysator durchführt.
